# EUROPEAN PATENT APPLICATION

(11) **EP 3 884 810 A1**
(43) Date of publication of application: **29.09.2021**
(21) Application number: 20464006.4
(22) Date of filing: 28.04.2020
(51) Int. Cl.: A47C 7/72, A47C 3/025, A61B 5/11, A61B 5/00, A47C 7/14

(54) **SYSTEM FOR MONITORING THE ACTIVITY OF A PERSON IN THE OFFICE**

(30) Priority: 23.03.2020 RO 202000146
(71) Applicant: Stefan cel Mare University of Suceava, 720229 Suceava, County Suceava (RO)
(72) Inventor: Milici, Laurentiu Dan, jud. Suceava (RO); Poienar, Mihaela, jud. Suceava (RO); Nitan, Ilie, jud. Suceava (RO); Ungureanu, Constantin, Suceava, jud. Suceava (RO); Grosu Oana, Vasilica, jud. Suceava (RO); Pocris, Marcel, Botosani, jud. Botosani (RO); Toader, Vasile-Eusebiu, jud. Suceava (RO); Medrihan, Dumitru-Nicolae, jud. Suceava (RO)

(57) **Abstract**

The system for monitoring the activity of a person in the office, according to the invention, consists mainly of a chair 1, which has a seating area made of two overlapping plates 2 and 3, separated by four springs 4, 4', 4", 4'", between which are placed, on the corners of the plates, four force sensors 5, 5', 5", 5"', an acquisition microsystem with microcontroller 6 that transmits the acquired data to a computer 8 to be saved and processed.

## Description

The invention relates to a system for monitoring the activity of a person in the office, based on the evaluation of the position of the monitored person during the period in which he carries out his activity sitting on a chair, but also during the rest period.

In order to develop systems for monitoring the activity of a person in the office, a solution is known (GUANTAO, L., Intelligent chair. Patent application no. CN107692614A, 2018-02-16), which consists mainly in the use of a pressure sensor placed in the seating area of the seat, to determine the user's weight, and according to this by means of a telescopic rod is made the adjustment of the seating position.

The disadvantages of the solution are related to the fact that a single pressure sensor is used placed on the seat area and thus errors occur in determining the position of the person on the seat.

The technical problem solved by the invention consists in the realization of a system for continuous monitoring of the activity of a person sitting on a chair based on the evaluation of the person's position according to his weight distribution on the surface of the chair on which he is seated.

The system for monitoring the activity of a person in the office, according to the invention, removes the disadvantages presented by the fact that it consists mainly of an acquisition system that takes data from four force sensors placed under the seating area of a chair.

The invention has the following advantages:
- the possibility to identify the type of activity carried out by the person sitting on the chair;
- the possibility to evaluate the level of involvement in activities of the person sitting on the chair;
   - the possibility of evaluating the time intervals during which the person sat on the chair;
   - accuracy of processed data;
   - constructive simplicity.

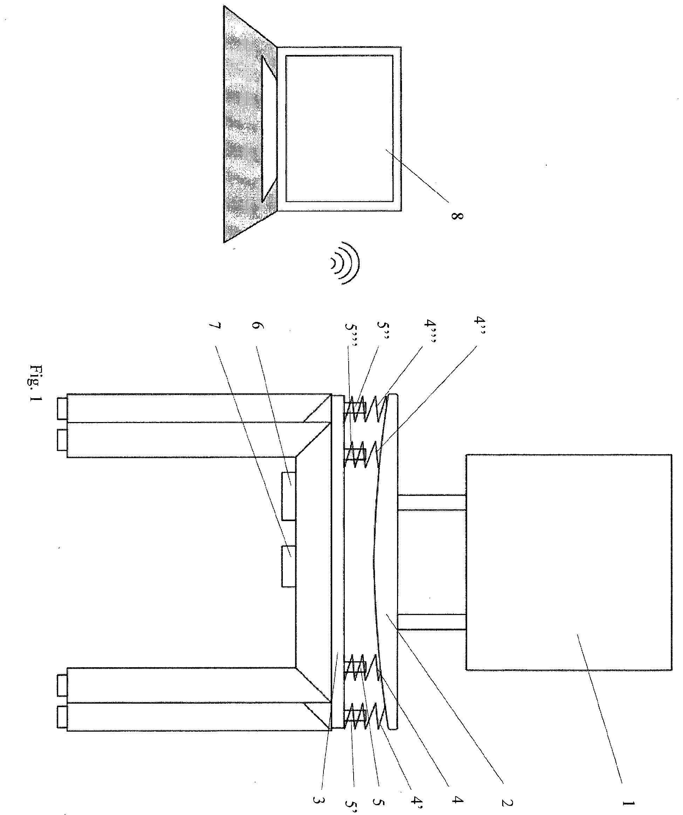

The following is an embodiment of the invention in connection with Figure 1 which is an overview of the system for monitoring the activity of a person in the office.

The system of monitoring the activity of a person in the office, according to the invention, consists mainly of a chair 1, which has the seating area made of two overlapping plates 2 and 3, separated by four springs 4, 4', 4", 4"', placed on the corners of the plates and four force sensors 5, 5', 5", 5"', connected to a acquisition microsystem with microcontroller 6 that allows to adapt the signals from the sensors by amplification and analog - digital conversion, a power supply 7 and the computer 8.

The system of monitoring the activity of a person in the office, takes through the sensors 5, 5', 5", 5"', informations about the distribution of body weight in the four points and the microcontroller 6 can determine by analyzing the variation of the signals in time from each sensor, by means of an algorithm adapted to the activity of the monitored person, the person's weight, the period that the person was in the chair, the position and activity (leaning in front of a paper sheet or a screen, lied back in a relaxed position, bent in the side to another person, with their feet resting on the floor or not, sudden movements, comfort level, continuous movements of the legs, etc.), information that is transmitted wirelessly, by the microcontroller 6 to a computer 8, where they are saved and can generate messages warning about changing weight over long periods of time, the need for relaxation breaks in case of long periods of working tasks, activities unsuitable for work tasks, states of overload, stress or anxiety of the monitored person.

The system of monitoring the activity of a person in the office, according to the invention, can be reproduced with the same performances and characteristics whenever necessary, which is an argument in favor of respecting the criterion of industrial applicability.

### References

[1]. GUANTAO, L., Intelligent chair. Pattent application no. CN107692614A, 2018-02-16.

## Claims

1. The system for monitoring the activity of a person in the office, **characterized in that** it consists mainly of a chair (1), which has a seating area made of two overlapping plates (2) and (3), separated by four springs (4), (4'), (4"), (4"'), between which are placed, on the corners of the plates, four force sensors (5), (5'), (5"), (5"'), connected to a acquisition microsystem with microcontroller (6) that allows the adaptation of the signals from the sensors by amplification and analogue-numerical conversion and also the acquisition of these signals, and a power supply (7), the acquired data being transmitted, wirelessly, to a computer (8), where they are saved, processed and can issue warnings.

2. The system for monitoring the activity of a person in the office according to claim 1, characterized through that depending on the weight distribution of the monitored person on the four sensors placed in the corners of the chair (1) can analyze, via a dedicated software application, the person's position on chair, its activity and position (front-back or left-right), sudden movements, comfort level, continuous movements.
